# EUROPEAN PATENT SPECIFICATION

(11) **EP 4 006 271 B1**
(45) Date of publication and mention of the grant of the patent: **16.10.2024**
(21) Application number: 21210472.3
(22) Date of filing: 25.11.2021
(51) Int. Cl.: E05B 1/00, A61L 2/18

(54) **DISPENSER FOR HANDLE**
FLÜSSIGKEITSSPENDER FÜR GRIFF
DIFFUSEUR POUR POIGNÉE

(30) Priority: 25.11.2020 DK PA202001333
(43) Date of publication of application: 01.06.2022
(73) Proprietor: Højsager, Jakob, 5250 Odense SV (DK); Winterskov, Dennis Bo, 5270 Odense N (DK)
(72) Inventor: Højsager, Jakob, 5250 Odense SV (DK); Winterskov, Dennis Bo, 5270 Odense N (DK)
(74) Representative: Larsen & Birkeholm A/S

(56) References cited:
- KR-A- 20160 063 660
- KR-B1- 101 370 608
- US-A1- 2010 294 806

## Description

The invention relates to a fluid dispenser for arrangement in handles of the type extending from a door and comprising a cylindrical door handle which ends in a free end, and wherein the cylindrical door handle comprises an opening to the fluid dispensed which dispenser comprises an end portion for fluid-tight assembly with the free end of the cylindrical door handle which end portion comprises a spring loaded button mechanism; a pump portion comprising a cylinder with an inlet channel and an outlet channel; a piston mutually adapted to the cylinder such that a movement of the piston in the cylinder causes transport of a fluid from inlet channel to outlet channel; a piston rod for connection between the spring loaded button mechanism and piston.

Today various mechanisms for dispensing disinfectant liquid on a handle are known, many operate with an electrical pump which may be activated by a button. The disadvantage of these mechanisms is that they require battery replacement in order to function for a long period of time. However, also pure mechanical systems are known which may dispense a disinfectant liquid onto the upper side of a door handle. Typically, these devices operate by activation of a pump mechanism when the handle is moved. Another type of mechanism is known from the Korean patent specification KR101370608, in which the mechanism itself is built into the door handle such that it may be activated by means of an internal spring mechanism that can be pushed by use of the handle. The disadvantage of these systems is that they are activated by movements of the handle which may mean that liquid is dispensed unintentionally. Such a situation may arise when users not wanting contact with the disinfectant liquid accidentally activate the disinfection mechanism. Typically, such users are allergic to the agents in the common liquids.

It is an object of the invention to provide a system remedying a disadvantage.

Another object of the invention is to provide an alternative fluid dispenser.

This is achieved with a fluid dispenser according to claim 1. In particular, the outlet channel is connected with the opening to the fluid dispensed and that distanced from the end portion, the pump portion is adapted to form a fluid-tight assembly with the inner sides of the cylindrical door handle such that the piston via the piston rod is moved in the cylinder by the spring loaded button mechanism and thereby generating an overpressure which transports a fluid from inlet channel to outlet channel.

Examples of embodiments are mentioned in the independent patent claims. An embodiment is now explained with reference to the figures in which:
- figure 1: shows a side view of a cylindrical door handle with dispenser in two situations,
- figure 2: shows a top view of a cylindrical door handle.

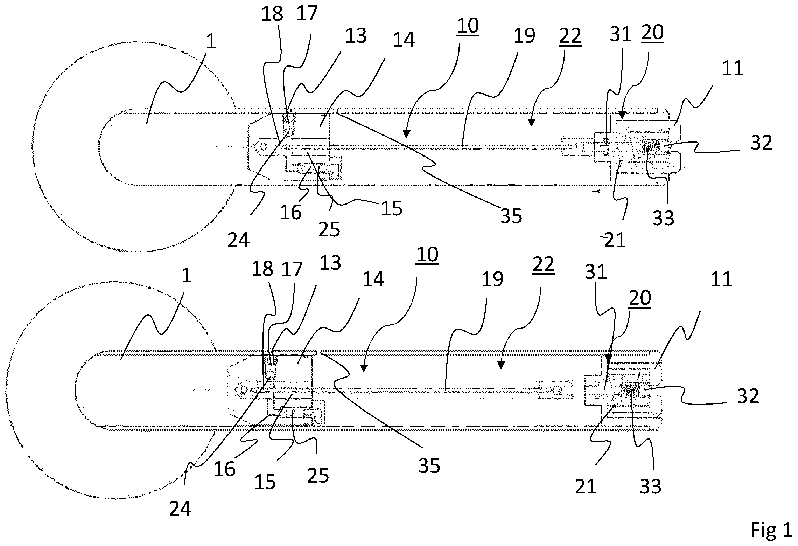

In figure 1, a cylindrical door handle is shown in 2 situations wherein the upper figure shows the situation in which a built-in fluid dispenser 10 is not activated and the lower figure shows a situation in which a built-in fluid dispenser 10 is activated via the spring loaded button mechanism 11 located in the free end of the cylindrical door handle. In the lower figure, the spring loaded button mechanism 11 is thus pushed in.

Fluid dispenser comprises an opening 13 for the fluid dispensed and it is arranged on the upper side of the cylindrical door handle. Fluid dispenser comprises an end portion 20 for fluid-tight assembly with the free end of the cylindrical door handle. The end portion comprises a spring loaded button mechanism 11.

Furthermore, the dispenser comprises a pump portion 14 with an inner cylinder 15 with an inlet channel 16 and an outlet channel 17. The end portion 20 and the pump portion 14 are adapted to the inside of the cylindrical door handle such that they form a tight sealing against the inner sides of the cylindrical door handle.

Thereby the cavity 22 between these portions may be used as reservoir for the fluid intended to be dispensed with the dispenser. However, for this it is required that the cylindrical door handle itself is designed in a tight material. Alternatively, it will be necessary to make the cylindrical door handle tight, it may for instance be done by lining the inner sides of the cylindrical door handle with a tight material for tight connection with end portion and pump portion.

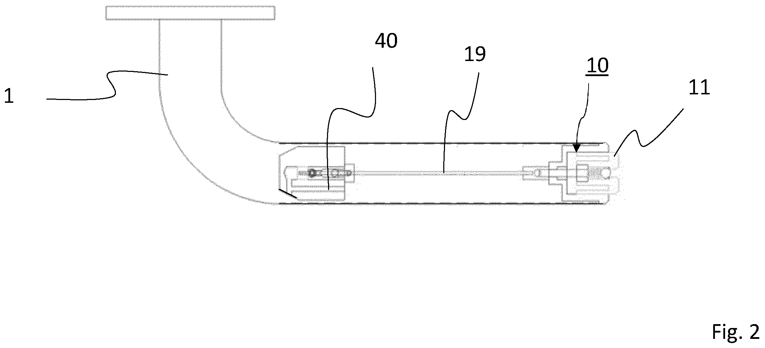

Inside the cylinder 15 of the pump portion, a piston 18 is arranged which is mutually adapted to the cylinder such that a movement of the piston 18 in the cylinder 15 causes transport of a fluid which is located in cavity 22 and cylinder 15 from inlet channel to outlet channel. A piston rod 19 connects between the spring loaded 11 button mechanism and piston 18. The dispensing occurs when a user pushes in the spring loaded 21 button mechanism. When this happens, the button 11 will act on the piston rod which in turn will press the piston 18 thereby creating an overpressure on its front side. This overpressure will in the depiction shown in figure 2 be equalized via the channel 40. As shown in figure 2, the channel 40 only serves this purpose, it may however be designed otherwise, for instance be equipped with valves and tubes to the outlet opening 13 such that this movement means that liquid is dispensed from it.

In the embodiment shown, the outlet channel 17 is connected with the opening 13 to the fluid dispensed.

In embodiments, fluid is moved towards the outlet channel when the spring loaded button mechanism is moved towards the inside of the cylindrical door handle. In the embodiment shown, fluid is moved towards the outlet channel when the spring loaded button mechanism is moved away from the inside of the cylindrical door handle. It will however naturally be easy for a person skilled in the art to provide a double acting mechanism wherein fluid thus is pumped out on the upper side of the cylindrical door handle when the push button is pressed in and once again when due to a spring mechanism the push button returns. This may possibly be achieved via a tube connection to channel 40 (shown in figure 2).

In the embodiment shown in figure 1, the fluid dispenser is designed such that the outlet channel 17 comprises a check valve allowing fluid to flow towards the opening 13 to the fluid dispensed. In this embodiment, the check valve is designed as a ball valve 24 which blocks when located at the bottom of the channel. The check valve may naturally be designed in other ways well known to the person skilled in the art.

In the same way, dispenser may be designed with the inlet channel comprising a check valve allowing fluid to flow into the pump portion, and also in this embodiment shown, the check valve is designed as a ball valve 25, however it may naturally be designed in other ways.

Fluid dispenser may also be designed such that the opening 13 itself towards the outer side of the cylindrical door handle comprises a check valve allowing fluid to flow towards the outer side (r) of the cylindrical door handle.

Fluid dispenser may be designed with a filler neck allowing fluid to be filled into the dispenser when the dispenser is positioned in a cylindrical door handle. In the embodiment shown in figure 1, this has been achieved by providing a channel 31 connecting the outer end of the button mechanism with the inner cavity 22. The button 31 is provided with a check valve in the form of a spring loaded 33 ball 32. In this embodiment, the filling itself takes place by a pointed filler neck pushing the ball 33 to the left (in the figure) while fluid is injected to the right. That is into the channel 31. To allow this, the door handle in the embodiment shown is provided with a vent valve 35 in the form of an opening. This is practical as the same opening may be used to allow air to enter the cylindrical door handle and thereby fill the volume decreasing as fluid is pumped out of the dispenser. This opening may naturally also be provided with a check valve allowing air to escape from the cylindrical door handle but not to enter. If this solution is used, it may be advantageous to provide the cylindrical door handle with an additional opening allowing air to enter and replace the volume decreasing when liquid is pumped out of the cylindrical door handle, and this opening may naturally also be provided with a check valve allowing this but blocking a fluid stream in opposite direction.

## Claims

1. Fluid dispenser (10) for arrangement in handles of the type extending from a door and comprising a cylindrical door handle which ends in a free end, and wherein the cylindrical door handle comprises an opening (13) to the fluid dispensed which dispenser comprises:
an end portion (20) for fluid-tight assembly with the free end of the cylindrical door handle which end portion comprises a spring loaded button mechanism (11);
a pump portion (14) comprising a cylinder (15) with an inlet channel (16) and an outlet channel (17); a piston (18) mutually adapted to the cylinder such that a movement of the piston in the cylinder causes transport of a fluid from inlet channel to outlet channel;
a piston rod (19) for connection between the spring loaded button mechanism and piston,
and wherein the outlet channel is connected with the opening to the fluid dispensed and that distanced from the end portion,
the pump portion is adapted to form a fluid-tight assembly with the inner sides of the cylindrical door handle such that the piston via the piston rod is moved in the cylinder by the spring loaded button mechanism and thereby generating an overpressure which transports a fluid from inlet channel to outlet channel.

2. Fluid dispenser according to claim 1, **characterised in that** fluid is moved towards the outlet channel when the spring loaded button mechanism is moved towards the inside of the cylindrical door handle.

3. Fluid dispenser according to claims 1-2, **characterised in that** fluid is moved towards the outlet channel when the spring loaded button mechanism is moved away from the inside of the cylindrical door handle.

4. Fluid dispenser according to claims 1-3, **characterised in that** the connection between the outlet channel and the opening to the fluid dispensed comprises a tube connection.

5. Fluid dispenser according to claims 1-4, **characterised in that** the outlet channel comprises a check valve (24) allowing fluid to flow towards the opening to the fluid dispensed.

6. Fluid dispenser according to claims 1-5, **characterised in that** the inlet channel comprises a check valve (25), allowing fluid to flow into the pump portion.

7. Fluid dispenser according to claims 1-6, **characterised in that** in the cylindrical door handle a cavity (10) for fluid is formed between end portion and pump portion when they are mounted in the cylindrical door handle and that this cavity is connected with the inlet channel such that the cavity may be used as reservoir for the fluid to be dispensed.

8. Fluid dispenser according to claims 1-7, **characterised in that** the opening (13) comprises a check valve allowing fluid to flow towards the outer side (r) of the cylindrical door handle.

9. Fluid dispenser according to claims 1-8, **characterised in that** the dispenser comprises a filler neck allowing fluid to be filled into the dispenser when the dispenser is arranged in a cylindrical door handle.

10. Fluid dispenser according to claims 1-9, **characterised in that** the filler neck is arranged in the end portion.

11. Fluid dispenser according to claims 1-10, **characterised in that** the filler neck is arranged in the spring loaded button mechanism.

12. Fluid dispenser according to claims 9-11, **characterised in that** the fluid dispenser comprises a valve device allowing air to escape from the cylindrical door handle when filling in fluid.

13. Fluid dispenser according to claims 1-12, **characterised in that** the fluid dispenser comprises a valve device allowing air to the cylindrical door handle when it is emptied of fluid.

## Patentansprüche

1. Fluidspender (10) zur Anordnung in Griffen der Art, die sich von einer Tür erstrecken und einen zylindrischen Türgriff umfassen, der in einem freien Ende endet, und wobei der zylindrische Türgriff eine Öffnung (13) zu dem abgegebenen Fluid umfasst, wobei der Spender Folgendes umfasst:
einen Endabschnitt (20) zur fluiddichten Montage mit dem freien Ende des zylindrischen Türgriffs, wobei der Endabschnitt einen federbelasteten Knopfmechanismus (11) umfasst;
einen Pumpenabschnitt (14), der einen Zylinder (15) mit einem Einlasskanal (16) und einem Auslasskanal (17) umfasst; einen Kolben (18), der derart gemeinsam mit dem Zylinder ausgelegt ist, dass eine Bewegung des Kolbens in dem Zylinder einen Transport eines Fluids von dem Einlasskanal zu dem Auslasskanal bewirkt;
eine Kolbenstange (19) zur Verbindung zwischen dem federbelasteten Knopfmechanismus und dem Kolben,
und wobei der Auslasskanal mit der Öffnung zu dem abgegebenen Fluid verbunden ist und von dem Endabschnitt beabstandet ist,
der Pumpenabschnitt dazu ausgelegt ist, derart eine fluiddichte Baugruppe mit den Innenseiten des zylindrischen Türgriffes zu bilden, dass der Kolben über die Kolbenstange in dem Zylinder durch den federbelasteten Knopfmechanismus bewegt wird und dadurch einem Überdruck generiert, der ein Fluid von dem Einlasskanal zu dem Auslasskanal transportiert.

2. Fluidspender nach Anspruch 1, **dadurch gekennzeichnet, dass** Fluid in Richtung des Auslasskanals bewegt wird, wenn der federbelastete Knopfmechanismus in Richtung des Inneren des zylindrischen Türgriffs bewegt wird.

3. Fluidspender nach Anspruch 1-2, **dadurch gekennzeichnet, dass** Fluid in Richtung des Auslasskanals bewegt wird, wenn der federbelastete Knopfmechanismus von dem Inneren des zylindrischen Türgriffs wegbewegt wird.

4. Fluidspender nach Anspruch 1-3, **dadurch gekennzeichnet, dass** die Verbindung zwischen dem Auslasskanal und der Öffnung zu dem abgegebenen Fluid eine Röhrenverbindung umfasst.

5. Fluidspender nach Anspruch 1-4, **dadurch gekennzeichnet, dass** der Auslasskanal ein Rückschlagventil (24) umfasst, das ermöglicht, dass Fluid in Richtung der Öffnung zu dem abgegebenen Fluid strömt.

6. Fluidspender nach Anspruch 1-5, **dadurch gekennzeichnet, dass** der Einlasskanal ein Rückschlagventil (25) umfasst, das ermöglicht, dass Fluid in den Pumpenabschnitt strömt.

7. Fluidspender nach Anspruch 1-6, **dadurch gekennzeichnet, dass** in dem zylindrischen Türgriff zwischen dem Endabschnitt und dem Pumpabschnitt ein Hohlraum (10) für Fluid gebildet ist, wenn sie in dem zylindrischen Türgriff montiert sind, und dass dieser Hohlraum derart mit dem Einlasskanal verbunden ist, dass der Hohlraum als Behälter für das abzugebende Fluid verwendet werden kann.

8. Fluidspender nach Anspruch 1-7, **dadurch gekennzeichnet, dass** die Öffnung (13) ein Rückschlagventil umfasst, das ermöglicht, dass Fluid in Richtung der Außenseite (r) des zylindrischen Türgriffs strömt.

9. Fluidspender nach Anspruch 1-8, **dadurch gekennzeichnet, dass** der Spender einen Einfüllstutzen umfasst, der ermöglicht, dass Fluid in den Spender eingefüllt wird, wenn der Spender in einem zylindrischen Türgriff angeordnet ist.

10. Fluidspender nach Anspruch 1-9, **dadurch gekennzeichnet, dass** der Einfüllstutzen in dem Endabschnitt angeordnet ist.

11. Fluidspender nach Anspruch 1-10, **dadurch gekennzeichnet, dass** der Einfüllstutzen in dem federbelasteten Knopfmechanismus angeordnet ist.

12. Fluidspender nach Anspruch 9-11, **dadurch gekennzeichnet, dass** der Fluidspender eine Ventilvorrichtung umfasst, die ermöglicht, dass beim Einfüllen von Fluid Luft aus dem zylindrischen Türgriff entweicht.

13. Fluidspender nach Anspruch 1-12, **dadurch gekennzeichnet, dass** der Fluidspender eine Ventilvorrichtung umfasst, die Luft zu dem zylindrischen Türgriff erlaubt, wenn dieser von Fluid entleert ist.

## Revendications

1. Diffuseur de fluide (10) pour un agencement dans des poignées du type se prolongeant à partir d'une porte et comprenant une poignée de porte cylindrique qui se termine par une extrémité libre, et dans lequel la poignée de porte cylindrique comprend une ouverture (13) pour le fluide diffusé, lequel diffuseur comprend :
une partie d'extrémité (20) pour un ensemble étanche aux fluides avec l'extrémité libre de la poignée de porte cylindrique, laquelle partie d'extrémité comprend un mécanisme de bouton chargé par ressort (11) ;
une partie de pompe (14) comprenant un cylindre (15) avec un canal d'entrée (16) et un canal de sortie (17) ; un piston (18) mutuellement adapté au cylindre de sorte qu'un mouvement du piston dans le cylindre entraîne le transport d'un fluide du canal d'entrée au canal de sortie ;
une tige de piston (19) pour une connexion entre le mécanisme de bouton chargé par ressort et le piston,
et dans lequel le canal de sortie est connecté à l'ouverture vers le fluide diffusé et éloigné de la partie d'extrémité,
la partie de pompe est conçue pour former un ensemble étanche aux fluides avec les côtés internes de la poignée de porte cylindrique de sorte que le piston via la tige de piston est déplacé dans le cylindre par le mécanisme de bouton chargé par ressort et générant ainsi une surpression qui transporte un fluide du canal d'entrée au canal de sortie.

2. Diffuseur de fluide selon la revendication 1, **caractérisé en ce que** le fluide est déplacé vers le canal de sortie lorsque le mécanisme de bouton chargé par ressort est déplacé vers l'intérieur de la poignée de porte cylindrique.

3. Diffuseur de fluide selon les revendications 1 et 2, **caractérisé en ce que** le fluide est déplacé vers le canal de sortie lorsque le mécanisme de bouton chargé par ressort est éloigné de l'intérieur de la poignée de porte cylindrique.

4. Diffuseur de fluide selon les revendications 1 à 3, **caractérisé en ce que** la connexion entre le canal de sortie et l'ouverture vers le fluide diffusé comprend une connexion de tube.

5. Diffuseur de fluide selon les revendications 1 à 4, **caractérisé en ce que** le canal de sortie comprend un clapet anti-retour (24) permettant au fluide de s'écouler vers l'ouverture vers le fluide diffusé.

6. Diffuseur de fluide selon les revendications 1 à 5, **caractérisé en ce que** le canal d'entrée comprend un clapet anti-retour (25), permettant au fluide de s'écouler dans la partie de pompe.

7. Diffuseur de fluide selon les revendications 1 à 6, **caractérisé en ce que** dans la poignée de porte cylindrique, une cavité (10) pour fluide est formée entre la partie d'extrémité et la partie de pompe lorsqu'elles sont montées dans la poignée de porte cylindrique et **en ce que** cette cavité est connectée au canal d'entrée de sorte que la cavité peut être utilisée comme réservoir pour le fluide à diffuser.

8. Diffuseur de fluide selon les revendications 1 à 7, **caractérisé en ce que** l'ouverture (13) comprend un clapet anti-retour permettant au fluide de s'écouler vers le côté externe (r) de la poignée de porte cylindrique.

9. Diffuseur de fluide selon les revendications 1 à 8, **caractérisé en ce que** le diffuseur comprend un goulot de remplissage permettant au fluide d'être introduit dans le diffuseur lorsque le diffuseur est disposé dans une poignée de porte cylindrique.

10. Diffuseur de fluide selon les revendications 1 à 9, **caractérisé en ce que** le goulot de remplissage est agencé dans la partie d'extrémité.

11. Diffuseur de fluide selon les revendications 1 à 10, **caractérisé en ce que** le goulot de remplissage est agencé dans le mécanisme de bouton chargé par ressort.

12. Diffuseur de fluide selon les revendications 9 à 11, **caractérisé en ce que** le diffuseur de fluide comprend un dispositif de vanne permettant à l'air de s'échapper de la poignée de porte cylindrique lors du remplissage de fluide.

13. Diffuseur de fluide selon les revendications 1 à 12, **caractérisé en ce que** le diffuseur de fluide comprend un dispositif de vanne permettant à l'air d'arriver à la poignée de porte cylindrique lorsqu'elle est vidée de fluide.
